# EUROPÄISCHE PATENTSCHRIFT

(11) **EP 0 569 812 B1**
(45) Veröffentlichungstag und Bekanntmachung des Hinweises auf die Patenterteilung: **13.03.1996**
(21) Anmeldenummer: 93107141.9
(22) Anmeldetag: 03.05.1993
(51) Int. Cl.: C07C 68/06, C07C 69/96

(54) **Verfahren zur kontinuierlichen Herstellung von Dialkylcarbonaten**
Process for the continuous production of dialkyle carbonates
Procédé de préparation continue de carbonates de dialkyle

(30) Priorität: 15.05.1992 DE 4216121
(43) Veröffentlichungstag der Anmeldung: 18.11.1993
(73) Patentinhaber: BAYER AG, D-51368 Leverkusen (DE)
(72) Erfinder: Wagner, Paul, Dr., W-4000 Düsseldorf 13 (DE); Mais, Franz-Josef, Dr., W-4000 Düsseldorf 1 (DE); Buysch, Hans-Josef, Dr., W-4150 Krefeld (DE); Langer, Reinhard, Dr., W-4150 Krefeld (DE); Klausener, Alexander, Dr., W-5000 Köln 1 (DE)

(56) Entgegenhaltungen:
- EP-A- 0 530 615
- US-A- 4 691 041

## Beschreibung

Die vorliegende Erfindung betrifft ein kontinuierliches Verfahren zur Herstellung von niederen Dialkylcarbonaten durch katalysierte Umesterung von Ethylenglykol- oder Propylenglykolcarbonat mit niederen Alkololen, wobei die Umsetzung des Alkylencarbonats mit einem Dialkylcarbonat enthaltenden Alkohol im Gegenstrom vorgenommen wird. Das Verfahren erfordert die Einführung von reinem Alkohol unterhalb der Einführungsstelle für den Dialkylcarbonat enthaltenden Alkohol.

Die Herstellung von Dialkylcarbonaten aus Ethylenglykolcarbonat und Alkoholen, bei der gleichzeitig das zugrundeliegende Ethylenglykol entsteht, ist bekannt und vielfach beschrieben worden. Fast alle Beschreibungen befassen sich jedoch mit der Katalyse dieser Umesterung, während die technische Realisierung dieses Verfahrens im Detail nicht beschrieben wird. In US 4.691.041 als einer der wenigen Quellen findet sich jedoch eine ausführliche Beschreibung eines solchen Verfahrens:
Ethylenglykolcarbonat und Methanol werden im Molverhältnis von 1:4 durch ein mit einem basischen Ionenaustauscher gefülltes Rohr bei etwa 100°C und etwa 7 bar gepumpt, wobei sich im günstigsten Fall das Umesterungsgleichgewicht einstellt, welches neben Methanol als Hauptkomponente und nicht umgesetztem Ethylenglykolcarbonat die Reaktionsprodukte Dimethylcarbonat und Ethylenglykol und zusätzlich Nebenprodukte, wie Polyglykole und Dimethylether, enthält.

Das beschriebene Reaktionsgemisch wird sodann in einer ersten Destillationsapparatur in die hochsiedenden Anteile Ethylenglykolcarbonat, Ethylenglykol und Polyglykole einerseits und die Leichtsieder Methanol, Dimethylcarbonat und Dimethylether andererseits aufgetrennt. In einer zweiten Destillation werden die Hochsieder in ein Ethylenglykol mit bis zu 10 % Ethylenglykolcarbonat und ein Polyglykole enthaltendes Ethylenglykolcarbonat aufgetrennt. Das Polyglykole enthaltende Ethylenglykolcarbonat fließt in die Umesterungsreaktion zurück, wobei die Gefahr der Aufkonzentrierung der hochsiedenden Nebenprodukte erwartet werden muß. Das Ethylenglykolcarbonat enthaltende Glykol wird in eine Hydrolyse eingespeist, in welcher das in das Ethylenglykol geratene Glykolcarbonat in Glykol und CO₂ aufgespalten wird und somit als Kohlensäurequelle verloren ist.

Aus der genannten Leichtsiederfraktion wird sodann in einer 3. Destillation, die unter erhöhtem Druck ausgeführt wird, ein Sumpf von reinem Dimethylcarbonat und ein an Dimethylcarbonat abgereichertes Methanol, welches zusätzlich Dimethylether enthält, erhalten. Das Dimethylcarbonat enthaltende Methanol wird ebenfalls in die Umesterungsreaktion zurückgeführt. Durch die Zurückführung von Carbonat enthaltendem Material wird die Raum-Zeit-Ausbeute an Umesterungsprodukt merklich eingeschränkt. Diese Einschränkung beträgt schätzungsweise 30 bis 40 %, verglichen mit der Erstfüllung der Umesterungsapparatur. Diese Einschränkung ist umso schmerzlicher, als ohnehin bestenfalls die Einstellung des Umesterungsgleichgewichts erwartet werden kann. Diese Einschränkungen sind für eine technische Durchführung außerordentlich hinderlich. Somit schien zwar die Rückführung eines Dialkylcarbonat enthaltenden Alkohols sehr wünschenswert, denn in einem solchen Falle brauchte eine weitgehende und damit energieaufwendige Auftrennung des Dialkylcarbonats und des zugrundeliegenden Alkohols nicht durchgeführt zu werden, eine solche Rückführung erschien aber nicht realisierbar. Die Vermeidung einer solchen aufwendigen Trennung von Carbonat und Alkohol ist in gleichem Maße für alle niederen Alkohole (C₁-C₄) wünschenswert; in besonderem Maße ist sie jedoch wünschenswert im System Dimethylcarbonat/Methanol, welches ein schwierig zu trennendes Azeotrop bildet.

In überraschend günstiger Weise ist die Benutzung eines Dialkylcarbonat enthaltenden Alkohols in einer technischen Realisierung dennoch möglich, wenn man in einer Kolonnenapparatur im Gegenstrom umestert, wobei dem im oberen Teil der Kolonne aufgegebenen Alkylencarbonat von unten her ein Dialkylcarbonat enthaltender Alkoholstrom entgegengeführt wird, wobei zusätzlich unterhalb der Einführung des Dialkylcarbonat enthaltenden Alkohols reiner Alkohol eingeführt wird.

Es wurde ein Verfahren zur kontinuierlichen Herstellung von Dialkylcarbonaten der Formel

(R¹O)₂CO (I),

in der R¹ geradkettiges oder verzweigtes C₁-C₄-Alkyl, bevorzugt Methyl oder Ethyl, besonders bevorzugt Methyl darstellt,
durch katalysierte Umesterung von Ethylenglykol- oder Propylenglykolcarbonat, bevorzugt von Ethylenglykolcarbonat, mit einem Alkohol der Formel

R¹OH (II),

in der R¹ die obige Bedeutung hat,
gefunden, das dadurch gekennzeichnet ist, daß die Umesterung in einer Kolonne im Gegenstrom so geführt wird, daß Ethylenglykol- oder Propylenglykolcarbonat in den oberen Teil der Kolonne und ein Dialkylcarbonat enthaltender Alkohol, dessen Dialkylcarbonatgehalt bei 0,2 bis 30 Gew.-% liegt, in den mittleren oder unteren Teil der Kolonne eingeführt werden und zusätzlich unterhalb der Einführung des Dialkylcarbonat enthaltenden Alkohols reiner Alkohol eingeführt wird.

Die Edukte für das erfindungsgemäße Verfahren sind demnach Ethylenglykolcarbonat oder Propylenglykolcarbonat, bevorzugt Ethylenglykolcarbonat einerseits und ein niederer Alkohol, wie Methanol, Ethanol, Propanol, Isopropanol, Butanol oder Isobutanol, andererseits, der mit dem zugehörigen Di-(C₁-C₄-alkyl)-carbonat vergesellschaftet ist. In einem solchen Gemisch beträgt der Anteil des Dialkylcarbonats 0,2 bis 30 Gew.-%, bevorzugt 1 bis 28 Gew.-%, besonders bevorzugt 3 bis 25 Gew.-%, bezogen auf die Gesamtmenge an Dialkylcarbonat und dem zugrundeliegenden Alkohol. Der einzusetzende Alkohol ist bevorzugt Methanol oder Ethanol, besonders bevorzugt Methanol; entsprechend ist das mit dem Alkohol vergesellschaftete Dialkylcarbonat das Dimethyl- bzw. Diethylcarbonat, bevorzugt das Dimethylcarbonat.

Das Alkylenglykolcarbonat wird erfindungsgemäß in den oberen Teil der Umesterungskolonne eingespeist. Der das zugehörige Dialkylcarbonat enthaltende Alkohol wird in den mittleren oder unteren Teil der Kolonne eingeführt; zusätzlich wird im erfindungsgemäßen Verfahren unterhalb der Einspeisungsstelle des das zugehörige Dialkylcarbonat enthaltenden Alkohols reiner Alkohol der gleichen Art eingeführt.

Der das zugehörige Dialkylcarbonat enthaltende Alkohol und der reine Alkohol werden in bevorzugter Weise gasförmig eingeführt und dem von oben herabrieselnden Alkylencarbonat entgegengeleitet.

Die Reaktionsprodukte des erfindungsgemäßen Verfahrens sind Ethylenglykol bzw. Propylenglykol, in bevorzugter Weise Ethylenglykol, welches als Sumpfprodukt gewonnen wird und einer weiteren Reinigung zugeführt wird. Desweiteren erhält man als Reaktionsprodukt am Kopf der Kolonne das gewünschte Dialkylcarbonat, im allgemeinen im Gemisch mit dem im Überschuß vorhandenen und nicht vollständig umgesetzten zugrundeliegenden Alkohol. Aus diesem als Kopfprodukt entnommenen Gemisch wird das gewünschte Dialkylcarbonat destillativ gewonnen. Bei dieser Gewinnung fällt der abzutrennende zugrundeliegende Alkohol nur bei hohen Anstrengungen, im speziellen Fall des Systems Dimethylcarbonat/Methanol wegen der Azeotropbildung nur bei äußerst hohen Anstrengungen in reiner Form an. Bei geringeren destillativen Aufwendungen erhält man dagegen stets ein Gemisch des abzutrennenden Alkohols mit dem zugehörigen Dialkylcarbonat.

Während der erfindungsgemäß das zugehörige Dialkylcarbonat enthaltende Alkohol aus einer beliebigen Quelle stammen kann (so beispielsweise aus der Herstellung von Alkyl-Arylcarbonat oder Diarylcarbonat, bei deren Reindarstellung ebenfalls ein Dialkyl/Alkohol-Gemisch anfällt), ist es erfindungsgemäß bevorzugt, einen solchen Dialkylcarbonat enthaltenden Alkohol einzusetzen, wie er bei der Reindarstellung der gewünschten Dialkylcarbonate im beschriebenen erfindungsgemäßen Verfahren unter Anlegung geringer destillativer Aufwendungen anfällt.

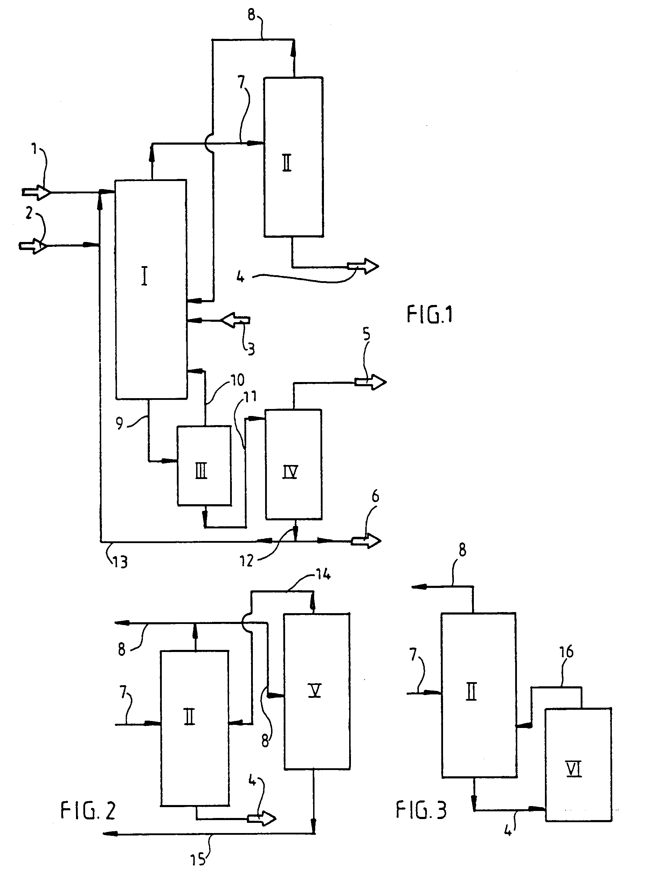

In Fig. 1 bis Fig. 5 werden verschiedene Aspekte des erfindungsgemäßen Verfahrens wie folgt dargestellt:
Fig. 1 zeigt als Apparate: eine Umesterungskolonne (I), eine Destillationskolonne (II) und Dünnschicht/Fallfilmverdampfer (111) und (IV). Fig. 1 zeigt weiterhin als Edukte: Alkylenglykolcarbonat (1), Katalysator (-Lösung) (2) und Alkohol (3). Fig. 1 zeigt noch weiterhin als Produkte: Dialkylcarbonat (4), Alkylenglykol (5) und zu entsorgende, katalysatorhaltige Hochsiederfraktion (6). Noch weiter zeigt Fig. 1 als interne Ströme: einen gasförmigen Strom eines Alkohol/Dialkylcarbonat-Gemisches (7), an Alkohol angereichertes Alkohol/Dialkylcarbonat-Gemisch (8), das nach (I) zurückgeführt wird, einen Glykol enthaltenden Sumpfstrom (9), Leichtsieder enthaltende Anteile (10) aus dem Sumpfstrom, wie noch vorhandener Alkohol und noch vorhandenes Dialkylcarbonat, die nach (I) zurückgeführt werden, katalysatorhaltiges rohes Alkylenglykol (11) und eine katalysatorhaltige Hochsieder enthaltende Fraktion (12), die zum Teil als solche (6) ausgeschleust wird und zum Teil im Sinne einer Katalysatorrückführung (13) auf den Kopf von (I) gegeben wird.

Fig. 1 zeigt demnach die bereits angesprochene Variante, in der der erfindungsgemäß einzusetzende, Dialkylcarbonat enthaltende Alkohol ein solcher ist, der bei der Reindarstellung des gewünschten Dialkylcarbonats anfällt. (I) kann eine mit dem Fachmann bekannten Einbauten (Glockenböden, Siebböden usw.) versehene oder mit Füllkörpern der dem Fachmann bekannten Art gepackte Kolonne sein. (II) ist eine Destillationskolonne, die in bevorzugter Weise bei erhöhtem Druck, beispielsweise bei 4 bis 15 bar, bevorzugt bei 6 bis 12 bar betrieben wird und die unter vereinfachten apparativen und energetischen Bedingungen (geringes Rücklaufverhältnis) betrieben werden kann. (III) und (IV) sind von allgemein bekannter Bauart, Die erfindungsgemäß einsetzbaren Umesterungskatalysatoren sind die dem Fachmann bekannten, beispielsweise Hydride, Oxide, Hydroxide, Alkoholate, Amide oder Salze von Alkalimetallen, wie Lithium, Natrium, Kalium, Rubidium und Cäsium, bevorzugt von Lithium, Natrium und Kalium, besonders bevorzugt von Natrium und Kalium (US-3 642 858, US-3 803 201, EP 1082). Für den Fall des Einsatzes der Alkoholate können diese erfindungsgemäß auch in situ durch Einsatz der elementaren Alkalimetalle und dem erfindungsgemäßen umzusetzenden Alkohol gebildet werden. Salze der Alkalimetalle können solche von organischen oder anorganischen Säuren sein, wie von Essigsäure, Propionsäure, Buttersäure, Benzoesäure, Stearinsäure, Kohlensäure (Carbonate oder Hydrogencarbonate), von Salzsäure, Bromwasserstoff- oder Iodwasserstoffsäure, Salpetersäure, Schwefelsäure, Fluorwasserstoffsäure, Phosphorsäure, Blausäure, Rhodanwasserstoff, Borsäure, Zinnsäure, C₁-C₄-Stannonsäuren oder Antimonsäuren. In bevorzugter Weise kommen als Verbindungen der Alkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt.

Solche Alkalimetallverbindungen (gegebenenfalls in situ gebildet aus den freien Alkalimetallen) werden in Mengen von 0,001 bis 2 Gew.-%, bevorzugt 0,005 bis 1,5 Gew.-%, besonders bevorzugt 0,01 bis 1,0 Gew.-%, bezogen auf das umzusetzende Reaktionsgemisch, eingesetzt.

Es ist erfindungsgemäß möglich, solchen Alkalimetallverbindungen gegebenenfalls komplexierende Stoffe zuzusetzen (EP 274 953). Beispielsweise seien genannt Kronenether wie Dibenzo-18-krone-6, Polyethylenglykole oder bicyclische stickstoffhaltige Kryptanden.

Solche Komplexiermittel werden in Mengen von 0,1 bis 200 mol-%, bevorzugt in 1 bis 100 mol-%, bezogen auf die Alkalimetallverbindung, eingesetzt.

Als Katalysatoren für das erfindungsgemäße Verfahren sind ferner Thallium-I- und Thallium-III-Verbindungen geeignet, wie die Oxide, Hydroxide, Carbonate, Acetate, Bromide, Chloride, Fluoride, Formiate, Nitrate, Cyanate, Stearate, Naphthenate, Benzoate, Cyclohexylphosphonate, Hexahydrobenzoate, das Cyclopentadienylthallium, Thalliummethylat, Thalliumethylat, bevorzugt Tl-(I)-oxid, Tl-(I)-hydroxid, Tl-(I)-carbonat, Tl-(I)-acetat, Tl-(III)-acetat, Tl-(I)-fluorid, Tl-(I)-formiat, Tl-(I)-nitrat, Tl-(I)-naphtenat und Tl-(I)-methylat (EP 1083). Die Mengen an Thalliumkatalysator sind nicht besonders kritisch. Sie betragen im allgemeinen 0,0001-10 Gew.-%, bevorzugt 0,001-1 Gew.-%, bezogen auf das gesamte Reaktionsgemisch.

Im erfindungsgemäßen Verfahren können weiterhin stickstoffhaltige Basen als Katalysatoren eingesetzt werden (US-4 062 884). Genannt seien beispielsweise sek. oder tert. Amine wie Triethylamin, Tributylamin, Methyldibenzylamin, Dimethylcyclohexylamin u.a..

Die erfindungsgemäß eingesetzten Mengen der stickstoffhaltigen Basen liegen von 0,01 bis 10 Gew.-%, bevorzugt von 0,1 bis 5 Gew.-%, besonders bevorzugt von 0,1 bis 1 Gew.-%, bezogen auf das gesamte Reaktionsgemisch.

Als Katalysatoren sind erfindungsgemäß ferner Verbindungen aus der Gruppe der Phosphine, Stibine, Arsine oder der zweiwertigen Schwefel- und Selen-Verbindungen sowie deren Oniumsalze einsetzbar (EP 180 387, US 4 734 519). Beispielhaft seien die folgenden genannt: Tributylphosphin, Triphenylphosphin, Diphenylphosphin, 1,3-Bis-(diphenylphosphino)propane, Triphenylarsin, Trimethylarsin, Tributylarsin, 1,2-Bis-(diphenylarsino)ethan, Triphenylantimon, Diphenylsulfid, Diphenyldisulfid, Diphenylselenid, Tetraphenylphosphoniumhalogenid (Cl, Br, J), Tetraphenylarsoniumhalogenid (Cl, Br, J), Triphenylsulfoniumhalogenid (Cl, Br) usw.

Die erfindungsgemäßen Einsatzmengen dieser Katalysatorengruppe liegen im Bereich von 0,1 bis 10 Gew.-%, bevorzugt von 0,1 bis 5 Gew.-%, besonders bevorzugt im Bereich von 0,1 bis 2 Gew.-%, bezogen auf das gesamte Reaktionsgemisch.

Weiterhin erfindungsgemäß einsetzbar sind Komplexe oder Salze des Zinns, Titans oder Zirkoniums (US 4 661 609). Beispiele solcher Systeme sind Butylstannonsäure, Zinnmethoxid, Dimethylzinn, Dibutylzinnoxid, Dibutylzinndilaurat, Tributylzinnhydrid, Tributylzinnchlorid, Zinn(II)ethylhexanoate, Zirkoniumalkoxide (Methyl, Ethyl, Butyl), Zirkonium(IV)halogenide (F, Cl, Br, J), Zirkoniumnitrate, Zirkoniumacetylacetonat, Titanalkoxide (Methyl, Ethyl, Isopropyl), Titanacetat, Titanacetylacetonat usw.

Die erfindungsgemäß einsetzbaren Mengen betragen 0,1 bis 10 Gew.-%, bevorzugt 0,5 bis 5 Gew.-%, bezogen auf das Gesamtgemisch.

Im erfindungsgemäßen Verfahren können weiterhin bifunktionelle Katalysatoren der Formel

[AₐX_{b}]ₘ·[B_{c}Y_{d}]ₙ (III),

eingesetzt werden. In diesen bifunktionellen Katalysatoren wird das molare Verhältnis der beiden in eckigen Klammern stehenden Komponenten durch die Indices m und n ausgedrückt. Diese Indices können unabhängig voneinander Werte von 0,001-1, bevorzugt 0,01-1, besonders bevorzugt 0,05-1 und ganz besonders bevorzugt 0,1-1 annehmen. Innerhalb der eckigen Klammern stehen neutrale Salze aus je einem Kation und einem Anion. Die Indices a und b stellen unabhängig voneinander ganze Zahlen von 1-5 dar; die Indices c und d bedeuten unabhängig voneinander ganze Zahlen von 1-3, wobei den Forderungen der Valenzen der Kationen und Anionen zur Bildung solcher neutraler Salze zu entsprechen ist. Des weiteren bedeuten in (III)
- A: das Kation eines Metalles, das der
dritten Periode und Gruppe IIa, der
vierten Periode und Gruppe IIa, IVa-VIIIa, Ib oder IIb, der
fünften Periode und Gruppe IIa, IVa-VIIa oder IVb bzw. der
sechsten Periode und Gruppe IIa-VIa des Periodensystems der Elemente in der Kurzperiodenform angehört.

Die in Frage kommenden Metalle für das Kation A entnimmt der Fachmann den üblichen Darstellungen des Periodensystems der Elemente (Mendelejew) in der Kurzperiodenform. In bevorzugter Weise handelt es sich bei A um das Kation eines der Metalle Mg, Ca, Sr, Ba, Zn, Cu, Mn, Co, Ni, Fe, Cr, Mo, W, Ti, Zr, Sn, Hf, V und Ta, in bevorzugter Weise um das Kation eines der Metalle Mg, Ca, Zn, Co, Ni, Mn, Cu und Sn. Außer den nicht komplexierten Kationen der genannten Metalle kommen auch kationische Oxokomplexe der genannten Metalle in Frage, wie beispielsweise Titanyl TiO⁺⁺ und Chromyl CrO₂⁺⁺.

Das zum Kation A gehörige Anion X ist das einer anorganischen oder organischen Säure. Eine solche anorganische oder organische Säure kann einbasisch oder zweibasisch oder dreibasisch sein. Solche Säuren und ihre Anionen sind dem Fachmann bekannt. Beispiele für Anionen einbasischer anorganischer oder organischer Säuren sind: Fluorid, Bromid, Chlorid, Iodid, Nitrat, das Anion einer Alkancarbonsäure mit 1-16 C-Atomen und Benzoat; Beispiele für Anionen zweibasischer anorganischer oder organischer Säuren sind: Sulfat, Oxalat, Succinat, Fumarat, Maleinat, Phthalat und weitere; Beispiele für dreibasische anorganische oder organische Anionen sind: Phosphat oder Citrat. Bevorzugte Anionen X im Katalysator der Formel (III) sind: Fluorid, Chlorid, Bromid, Iodid, Sulfat, Nitrat, Phosphat, Formiat, Acetat, Propionat, Oxalat, Butyrat, Citrat, Succinat, Fumarat, Maleinat, Benzoat, Phthalat, Decanoat, Stearat, Palmitat, und Laurinat. Besonders bevorzugte Anionen X sind: Chlorid, Bromid, Iodid, Acetat, Laurinat, Stearat, Palmitat, Decanoat, Nitrat und Sulfat.

Als Kation B in den Katalysatoren der Formel (III) kommt eines aus der Gruppe der Alkali- oder Erdalkalikationen, der quaternären Ammonium-, Phosphonium-, Arsonium- oder Stibonium-Kationen und der ternären Sulfonium-Kationen in Frage.

Als (Erd)Alkalimetallkationen seien hierbei genannt: das Lithium-, Natrium-, Kalium-, Rubidium-, Cäsium-, Magnesium-, Calcium-, Strontium- und Bariumkation, bevorzugt die genannten Alkalimetallkationen, besonders bevorzugt das Natrium- und das Kaliumkation.

In bevorzugter Weise kommen als Kationen B solche der Formeln 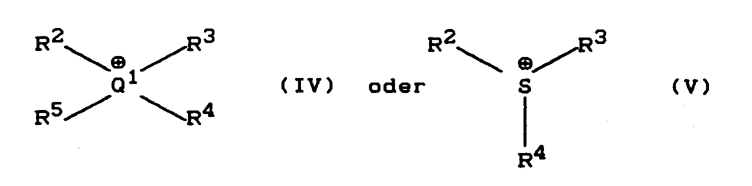
in Frage, worin
- Q¹: für N, P, As oder Sb steht und
- R², R³, R⁴ und R⁵: unabhängig voneinander geradkettiges oder verzweigtes C₁-C₁₈ oder C₇-C₁₂-Aralkyl sind und einer der Reste R²-R⁵ auch C₆-C₁₂ sein kann.

In besonders bevorzugter Weise ist B ein Kation der Formel 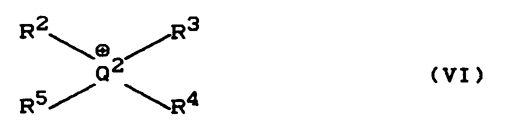
worin,
- Q²: für N oder P, bevorzugt für N steht.

In ganz besonders bevorzugter Weise treten im Rahmen der Formeln (IV) bzw. (VI) an die Stelle der Reste R², R³, R⁴ und R⁵ die Reste R¹², R¹³, R¹⁴ bzw. R¹⁵, die unabhängig voneinander geradkettiges oder verzweigtes C₁-C₁₂-Alkyl oder C₇-C₈-Aralkyl bedeuten und einer der Reste R¹² bis R¹⁵ auch Phenyl sein kann. In weiterhin ganz besonders bevorzugter Weise treten an die Stelle der Reste R¹², R¹³, R¹⁴ bzw. R¹⁵, die Reste R²², R²³, R²⁴ bzw. R²⁵, die unabhängig voneinander C₁-C₈-Alkyl oder Benzyl bedeuten und einer der Reste R²² bis R²⁵ auch Phenyl sein kann.

Geradkettiges oder verzweigtes C₁-C₁₈-Alkyl ist beispielsweise Methyl, Ethyl, Propyl, Isopropyl, Butyl, Isobutyl, Hexyl, Octyl, Dodecyl, Hexadecyl oder Octadecyl. Bevorzugtes Alkyl hat 1-12 C-Atome, besonders bevorzugtes Alkyl hat 1-8 C-Atome.

C₇-C₁₂-Aralkyl ist beispielsweise Benzyl, Phenylethyl, Phenylpropyl, Naphthylmethyl oder Naphthyl-ethyl; bevorzugtes Aralkyl ist Benzyl oder Phenylethyl, ganz besonders bevorzugtes Aralkyl ist Benzyl.

C₆-C₁₂-Aryl ist beispielsweise Phenyl, Naphthyl oder Biphenylyl, bevorzugt Phenyl.

Das Anion Y im Katalysator der Formel (III) ist ein Halogenidion, wie Fluorid, Chlorid, Bromid oder Iodid, bevorzugt Bromid oder Iodid, besonders bevorzugt Iodid. Es kann jedoch auch die Bedeutung von anderen unter X genannten Anionen besitzen, wenn im konkreten Fall das Anion X Bromid oder Iodid ist.

Der bifunktionelle Katalysator der Formel (III) wird in einer Menge von 0,005-5 Gew.-%, bevorzugt 0,01-3 Gew.-%, besonders bevorzugt 0,01-1 Gew.-%, bezogen auf das gesamte Umesterungsgemisch, eingesetzt.

Diese Katalysatormengen unterscheiden sich teilweise von den in der Literatur genannten Mengen. Besonders überraschend ist, daß im erfindungsgemäßen Verfahren relativ hohe Konzentrationen an den wirksamen Katalysatoren auf der Basis von Alkaliverbindungen eingesetzt werden können, ohne daß es dabei zu den ausbeutemindernden und die Reaktionsführung behindernden Entwicklungen von CO₂ und zur Bildung von Polyolen kommt, wie dies beispielsweise aus DE-OS 2 740 243 und der dort zitierten Literatur und aus DE-OS 2 740 251 bekannt ist. Auch dies ist eine überraschende Besonderheit des erfindungsgemäßen Verfahrens.

Solche Katalysatoren können homogen gelöst auf den Kopf der Kolonne gegeben werden, wobei als Lösungsmittel Alkylenglykolcarbonat, Alkylenglykol, Alkohol oder Dialkylcarbonat, also systemeigene Lösungsmittel, zur Anwendung gelangen. Es ist selbstverständlich möglich, nicht lösliche Umesterungskatalysatoren einzusetzen, die auf den Zwischenböden oder inmitten der Füllkörper von (I) angeordnet sind. In einem solchen Fall kann die Dosierung eines gelösten Katalysators über (2) entfallen. Geeignete heterogene Katalysatoren sind beispielsweise:
Ionentauscherharze mit funktionellen Gruppen aus tert. Aminen, quartären Ammoniumgruppen, wobei als Gegenionen Hydroxid, Chlorid oder Hydrogensulfat beispielsweise genannt seien, Sulfonsäuregruppen oder Carboxylgruppen, wobei für beide als Gegenionen Wasserstoff, Alkalimetalle oder Erdalkalimetalle beispielhaft genannt seien. Diese funktionellen Gruppen können entweder direkt oder über inerte Ketten an das Polymer gebunden sein (US 4 062 884, US 4 691 041, JA 63/238 043, EP 298 167). Weiterhin genannt seien Alkali- oder Erdalkalisilikate, imprägniert auf Siliciumdioxidträgern, sowie Ammonium-ausgetauschte Zeolithe.

Fig. 2 zeigt eine Ergänzung und Verbesserung der Auftrennung des Kopfstromes (7) von (I). Hierbei wird, wie in Fig. 1 dargestellt, zunächst in (II) reines Dialkylcarbonat (4) abgetrennt und ein Dialkylcarbonat enthaltender Alkohol (8) gasförmig entnommen. (8) wird nunmehr in Abwandlung von Fig. 1 nur zum Teil (beispielsweise 30 bis 80 %, bevorzugt 40 bis 70 %, des gesamten Stromes) direkt nach (I) zurückgeleitet, während der Rest in eine zweite Destillationskolonne (V) etwa mittig eingeführt wird und dort in einen nahezu reinen Alkohol darstellenden Sumpfstrom (15) und einen nahezu das gesamte Dialkylcarbonat neben weiterem Alkohol enthaltenden Kopfstrom (14) aufgeteilt. Während (14) zur weiteren Gewinnung von Dialkylcarbonat nach (II) zurückgeleitet wird, wird (15) in bevorzugter Weise als der erfindungsgemäß einzusetzende reine Alkohol in (1) eingesetzt und stellt dort einen Teil von (3) dar.

Fig. 3 zeigt einen noch weiteren Aspekt des erfindungsgemäßen Verfahrens, nämlich die Verknüpfung mit der Herstellung von Arylalkylcarbonat oder Diarylcarbonat, welches durch Umsetzung von Dialkylcarbonat mit einem Phenol gewonnen wird und bei welchem ein Gemisch aus abgespaltenem Alkohol und nicht vollständig umgesetztem Dialkylcarbonat (16) entsteht. Für den Fall, daß (16) mit seinem Gehalt an Dialkylcarbonat im erfindungsgemäßen Bereich von (8) liegt, kann ein solcher Strom anstelle von (8) erfindungsgemäß eingesetzt werden. Gemäß Fig. 3 wird er jedoch zur Rückgewinnung des Dialkylcarbonats und zur weitgehenden Abtrennung des zugrundeliegenden Alkohols nach (II) zurückgeführt.

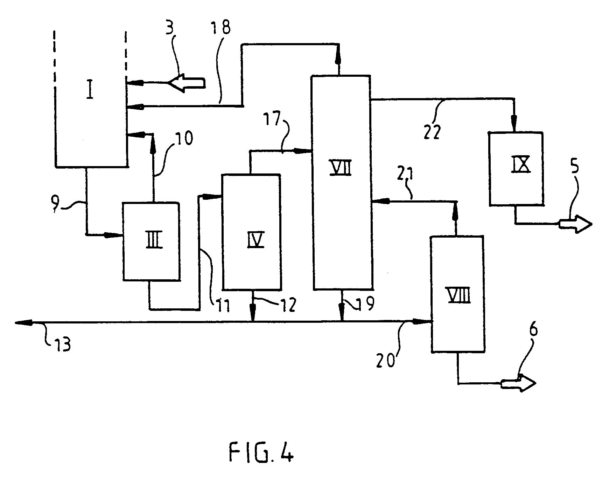

Fig. 4 schließlich zeigt eine weitere bevorzugte Variante in der Behandlung des Sumpfstromes (9) zur Gewinnung des erfindungsgemäß anfallenden Alkylenglykols und zur Entsorgung der Hochsiederfraktion (6). Hierbei wird aus (IV) zunächst ein Glykolstrom (17) entnommen, der in einer weiteren Destillationskolonne (VII) auf gereinigtes Glykol (22) aufgearbeitet wird, das bei Bedarf in einem weiteren Reinigungsschritt (IX) (nochmalige Destillation, Extraktion oder ähnliches) auf reines Glykol (5) aufgearbeitet werden kann. (22) wird im oberen Bereich von (VII) als Seitenstrom entnommen. Ein in (VII) anfallender Leichtsiederstrom (18) wird nach (I) zurückgeführt. Der in (VII) anfallende Hochsiederstrom (19) kann teilweise, genauso wie (12), in die Rückführungsleitung (13) gegeben werden, wird aber mindestens teilweise in (VIII) bezüglich seines Gehalts an Hochsiedern aufkonzentriert, wobei weiteres Glykol (21) anfällt. Das aufkonzentrierte Sumpfprodukt von (VIII) wird als (6) ausgeschleust.

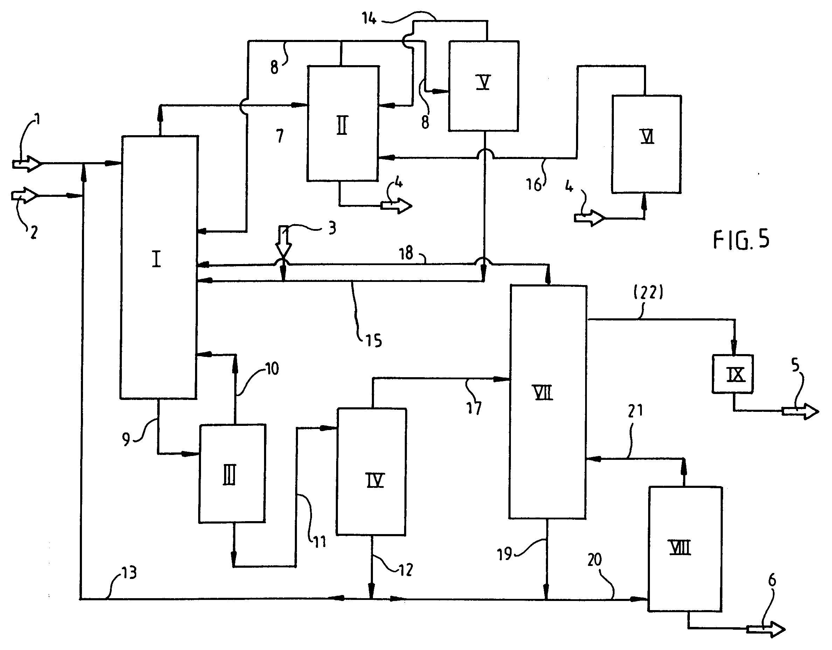

Fig. 5 zeigt eine integrierte Verfahrensvariante, in der die bevorzugten Elemente von Fig. 2, Fig. 3 und Fig. 4 in Fig. 1 integriert worden sind. Alle Apparate- und Stoffströme sind in Fig. 5 mit den gleichen Bezugszeichen markiert wie in Fig. 1 bis Fig. 4.

Die Umesterung in (1) wird bei 0,5 bis 5 bar, bevorzugt 0,8 bis 1,5 bar, besonders bevorzugt bei Normaldruck und bei einer Temperatur von 40 bis 150°C, bevorzugt bei 45 bis 130°C, durchgeführt.

Der erfindungsgemäß einzuspeisende reine Alkohol (3) bzw. zusätzlich (15) beträgt das 0,2 bis 5-fache des Dialkylcarbonatanteils in dem über (8) einzuführenden, ein Dialkylcarbonat enthaltenden Alkohol.

Der aus (I) entnommene Kopfstrom (7) hat beispielsweise im Falle von Methanol/Dimethylcarbonat die Zusammensetzung des Azeotrops mit 70 Gew.-% Methanol und 30 Gew.-% Dimethylcarbonat oder hat höhere Anteile an Dimethylcarbonat.

Der als (3) einzusetzende Alkohol kann auch aus einem Alkohol/Dialkylcarbonat-Gemisch gewonnen werden, aus welchem durch Pervaporation, Dampfpermeation oder Zweidruckdestillation das Dialkylcarbonat abgetrennt wurde.

Das erfindungsgemäße Verfahren hat gegenüber dem aus US 4.691.041 bekannten Stand der Technik folgende Vorteile:
a) Die Gleichgewichtseinstellung erfolgt rasch und führt durch ständig sich ändernde Stoffzusammensetzung zu sehr hohen Umsätzen und sehr hohen Selektivitäten.
b) Durch die praktische vollständige Umesterung des Alkylencarbonats entsteht ein von Alkylencarbonat praktisch freies Alkylenglykol; die vom Stand der Technik (US' 041) her bekannten Verluste von Alkylencarbonat durch nachträgliche Hydrolyse werden hierbei vermieden.
c) Infolge kurzer Verweilzeiten ist die Bildung von Nebenprodukten, wie die Bildung von Polyglykolen, auf Mengen unterhalb von 1 %, bezogen auf die Gesamtmenge an Alkylenglykol, begrenzt.
d) Das erfindungsgemäße Verfahren kommt mit wenigen Apparaten aus, die sich aus standardisierten Typen rekrutieren.
e) In der bevorzugten Ausführungsform gemäß Fig. 1 bis Fig. 5 werden viele interne Ströme nicht kondensiert, sondern als dampfförmige Ströme geführt. Damit werden die zu destillierenden Stoffmengen verkleinert und eine energetisch günstige Fahrweise geschaffen.
f) Eine verfahrenstechnisch günstige Kopplung mit der Herstellung von Alkyl-Arylcarbonaten oder Diarylcarbonaten ist möglich.

### Beispiel

In einer Apparateanordnung gemäß Fig. 5 wurde eine Gegenstromumesterungskolonne (I) mit Abtriebsteil und Verstärkerteil unter Anlegung eines Temperaturgradienten so temperiert, daß die Sumpftemperatur bei etwa 120°C und die Kopftemperatur bei etwa 50°C lag. Über (1) dosierte man 367 g/h Ethylenglykolcarbonat. Über (8) dosierte man 872 g/h eines Gemisches aus 80 Gew.-% Methanol und 20 Gew.-% Dimethylcarbonat. In den unteren Teil der Kolonne, jedoch oberhalb des Abtriebsteils dosierte man über (3) 270 g/h Methanol und über (15) weitere 130 g/h Methanol. Weiterhin wurden auf den Kopf von (I) über (13) 37 bis 38 g/h eines rückgeführten, 4 Gew.-% Katalysator (KOH) enthaltenden Sumpfes und 1,2 g/h frisches Kaliumhydroxid über (2) dosiert. Soweit diese eindosierten Stoffströme interne Stoffströme darstellten, wurden sie den in Fig. 5 gezeigten Apparaten entnommen.

Die Methanol enthaltenden eindosierten Stoffströme stiegen dampfförmig in der Kolonne nach oben, dem herabfließenden flüssigen und den Katalysator enthaltenden Ethylenglykolcarbonat entgegen, wobei die Umesterung zu Dimethylcarbonat und Ethylenglykol erfolgte. Am Kopf von (I) entnahm man 1380 g/h eines Gemisches (7) aus 60 Gew.-% Methanol und 40 Gew.-% Dimethylcarbonat, welches einer Bodenkolonne (II) in der Mitte aufgegeben und bei einem Druck von 10 bar in ein Gemisch aus etwa 80 Gew.-% Methanol und 20 Gew.-% Dimethylcarbonat und 378 g/h Dimethylcarbonat (4) aufgetrennt wurde. Vom dampfförmig entnommenen Gemisch (8) wurden die genannten 872 g/h nach (I) zurückgeführt, während der restliche Teil von (8) in der Kolonne (V) weiter aufgetrennt wurde; hierbei erhielt man ein Dimethylcarbonat enthaltendes Kopfprodukt, welches nach (II) zurückgeführt wurde und 130 g/h im wesentlichen aus Methanol bestehendes Sumpfprodukt (15), welches nach (I) zurückgeführt wurde.

Das Sumpfprodukt (9) von (I), das im wesentlichen aus Ethylenglykol, geringen Mengen Leichtsiedern (Methanol und Dimethylcarbonat), Hochsiedern, wie Diethylenglykol und dem Katalysator bestand, gelangte in einen Fallfilmverdampfer (III), aus welchem 38 g/h Leichtsieder (10) nach (I) zurückgeführt wurden. 375 g/h Sumpfprodukt (11) gelangten in einen weiteren Fallfilmverdampfer (IV), dem über seinen Sumpf 75 g/h aufkonzentrierte Katalysatorlösung (12) entnommen wurde, die etwa zur Hälfte über (13) nach (I) zurückgeführt und zur anderen Hälfte in einen Dünnschichtverdampfer mit Trennaufsatz (VIII) eindosiert wurde. 302 g/h Dampfphase (17) aus (IV) wurden in die Kolonne (VII) eindosiert. In (VII) wurden nochmals 77 g/h Leichtsieder (18) abgetrennt, die nach (I) zurückgeführt wurden. Aus dem oberen Teil von (VII) wurden im Seitenabzug 255 bis 256 g/h Glykol (22) entnommen, die je nach Reinheitsanforderungen in (IX) weiter auf hochreines Glykol (5) mit einer Menge von 255 g/h weiter behandelt werden konnten. Der Sumpfablauf (19) von (VII) in einer Menge von 58 bis 59 g/h wurde gemeinsam mit der Hälfte des Sumpfablaufs (12) von (IV) einem Dünnschichtverdampfer (VIII) zugeführt, dessen Destillat (21) mit einer Menge von 91 g/h in den unteren Teil von (VII) eingespeist wurde. Der aufkonzentrierte Sumpf (6) von (VIII) mit allen Hochsiedern und einem Teil des Katalysators wurde der Entsorgung zugeführt.

## Patentansprüche

1. Verfahren zur kontinuierlichen Herstellung von Dialkylcarbonat der Formel
(R¹O)₂CO,
in der R¹ geradkettiges oder verzweigtes C₁-C₄-Alkyl, bevorzugt Methyl oder Ethyl, besonders bevorzugt Methyl darstellt,
durch katalysierte Umesterung von Ethylenglykol- oder Propylenglykolcarbonat, bevorzugt von Ethylenglykolcarbonat, mit einem Alkohol der Formel
R¹OH,
in der R¹ die obige Bedeutung hat,
dadurch gekennzeichnet, daß die Umesterung in einer Kolonne im Gegenstrom so geführt wird, daß Ethylenglykol- oder Propylenglykolcarbonat in den oberen Teil der Kolonne und ein Dialkylcarbonat enthaltender Alkohol, dessen Dialkylcarbonatgehalt bei 0,2 bis 30 Gew.-% liegt, in den mittleren oder unteren Teil der Kolonne eingeführt werden und zusätzlich unterhalb der Einführung des Dialkylcarbonat enthaltenden Alkohols reiner Alkohol eingeführt wird.

2. Verfahren nach Anspruch 1, dadurch gekennzeichnet, daß der Dialkylcarbonat enthaltende Alkohol aus der destillativen Trennung des aus der Umesterung als Kopfstrom entnommenen Dialkylcarbonat/Alkohol-Gemisches in Dialkylcarbonat und einen Dialkylcarbonat enthaltenden Alkohol stammt.

3. Verfahren nach Anspruch 2, dadurch gekennzeichnet, daß die destillative Trennung bei 4 bis 15 bar, bevorzugt bei 6 bis 12 bar vorgenommen wird.

4. Verfahren nach Anspruch 1, dadurch gekennzeichnet, daß der reine Alkohol das 0,2 bis 5-fache des Dialkylcarbonatanteils im Dialkylcarbonat enthaltenden Alkohol darstellt.

5. Verfahren nach Anspruch 1, dadurch gekennzeichnet, daß die Umesterung bei einer Temperatur von 40 bis 150°C, bevorzugt bei 45 bis 130°C und einem Druck von 0,5 bis 2 bar, bevorzugt 0,9 bis 1,2 bar durchgeführt wird.

6. Verfahren nach Anspruch 1, dadurch gekennzeichnet, daß Dimethylcarbonat durch Umesterung von Ethylenglykolcarbonat mit Methanol hergestellt wird.

7. Verfahren nach Anspruch 2, dadurch gekennzeichnet, daß der bei der destillativen Trennung des aus der Umesterung als Kopfstrom entnommenen Dialkyl/Alkohol-Gemisches erhaltene weitere Kopfstrom zu 30 bis 80 Vol-%, bevorzugt zu 40 bis 70 Vol-% in die Umesterung zurückgeführt wird, während der Rest einer weiteren Destillation zugeführt wird, deren Sumpfprodukt im wesentlichen aus dem Alkohol besteht und in den unteren Teil der Umesterungskolonne zurückgeführt wird, während das Kopfprodukt dieser zweiten Kolonne in die erste Trennkolonne (II) zurückgeführt wird.

8. Verfahren nach Anspruch 2, dadurch gekennzeichnet, daß ein aus der Herstellung von Alkyl-Arylcarbonat oder Diarylcarbonat stammender Dialkylcarbonat/Alkohol-Strom in die Trennkolonne (II) eingespeist wird.

9. Verfahren nach Anspruch 1, dadurch gekennzeichnet, daß das nach Durchlaufen der Fallstrom-/Dünnschichtverdampfer (III) und (IV) erhaltene rohe Glykol (17) einer Glykol-Reindestillation in einer Kolonne (VII) zugeführt wird, an deren Kopf eine Leichtsiederfraktion (18) entnommen und in die Umesterungskolonne (I) zurückgeführt wird, unterhalb deren Kopf ein Seitenabzug (22) von Reinglykol entnommen wird, das in einer nachgeschalteten Reinigungsstufe (IX) weiter gereinigt werden kann, und deren Sumpf (19) gemeinsam mit einem Teil des Sumpfes (12) von (IV) einer Aufkonzentrierung in (VIII) zugeführt wird, deren Kopfstrom (21) der Glykol-Kolonne (VII) zugeführt und deren Sumpfprodukt (6) einer Entsorgung der Hochsieder und eines Teils des Katalysators zugeführt wird.

## Claims

1. Process for the continuous preparation of a dialkyl carbonate of the formula
(R¹O)₂CO,
in which R¹ signifies straight-chain or branched C₁-C₄-alkyl, preferably methyl or ethyl, particularly preferably methyl,
by catalysed reesterification of ethylene glycol carbonate or propylene glycol carbonate, preferably of ethylene glycol carbonate, with an alcohol of the formula
R¹OH,
in which R¹ has the above meaning,
characterised in that the reesterification is carried out in a column in counter-current in such a manner that ethylene glycol carbonate or propylene glycol carbonate is introduced into the upper part of the column and a dialkyl carbonate-containing alcohol, whose dialkyl carbonate content is 0.2 to 30 % by weight, is introduced into the central or lower part of the column and pure alcohol is additionally introduced below the introduction of the dialkyl carbonate-containing alcohol.

2. Process according to Claim 1, characterised in that the dialkyl carbonate-containing alcohol originates from the separation by distillation of the dialkyl carbonate/alcohol mixture withdrawn as head stream from the reesterification into dialkyl carbonate and a dialkyl carbonate-containing alcohol.

3. Process according to Claim 2, characterised in that the separation by distillation is carried out at 4 to 15 bar, preferably at 6 to 12 bar.

4. Process according to Claim 1, characterised in that the pure alcohol signifies 0.2 to 5 times the dialkyl carbonate fraction in the dialkyl carbonate-containing alcohol.

5. Process according to Claim 1, characterised in that the reesterification is carried out at a temperature of 40 to 150°C, preferably at 45 to 130°C and a pressure of 0.5 to 2 bar, preferably 0.9 to 1.2 bar.

6. Process according to Claim 1, characterised in that dimethyl carbonate is prepared by reesterification of ethylene glycol carbonate with methanol.

7. Process according to Claim 2, characterised in that 30 to 80 % by volume, preferably 40 to 70 % by volume, of the further head stream, obtained in the separation by distillation of the dialkyl/alcohol mixture withdrawn as a head stream from the reesterification, is returned to the reesterification, while the remainder is fed to a further distillation, the bottom product of which is essentially composed of the alcohol and is returned to the lower part of the reesterification column, while the head product of this second column is returned to the first separation column (II).

8. Process according to Claim 2, characterised in that a dialkyl carbonate/alcohol stream originating from the preparation of alkyl-aryl carbonate or diaryl carbonate is supplied to the separation column (II).

9. Process according to Claim 1, characterised in that the crude glycol (17) obtained after passage through the falling film/thin film evaporators (III) and (IV) is fed to a glycol purifying distillation in a column (VII) at the head of which a low-boiler fraction (18) is withdrawn and returned to the reesterification column (I), below the head of which a side stream (22) of pure glycol is withdrawn, which can be further purified in a purification stage (IX) connected downstream, and the bottom phase (19) of which, together with a part of the bottom phase (12) of (IV), is fed to a concentration in (VIII), the head stream (21) of which is fed to the glycol column (VII) and the bottom product (6) of which is fed to disposal of the high boilers and of part of the catalyst.

## Revendications

1. Procédé pour la préparation continue d'un carbonate de dialkyle de formule
(R¹O)₂CO,
dans laquelle R¹ représente un groupe alkyle à chaîne droite ou ramifiée en C₁ à C₄, de préférence un groupe méthyle ou éthyle, plus spécialement un groupe méthyle,
par transestérification catalysée du carbonate d'éthylèneglycol ou de propylèneglycol, de préférence le premier nommé, par un alcool de formule
R¹OH,
dans laquelle R¹ a les significations indiquées ci-dessus,
caractérise, en ce que la transestérification est réalisée dans une colonne à contrecourant dans laquelle on envoie, à la partie supérieure, le carbonate d'éthylèneglycol ou de propylèneglycol et, dans la partie médiane ou dans la partie inférieure de la colonne, un alcool contenant du carbonate de dialkyle, à une teneur en carbonate de dialkyle de 0,2 à 30 % en poids, et en outre, au-dessous du point d'introduction de l'alcool contenant du carbonate de dialkyle, on introduit l'alcool pur.

2. Procédé selon revendication 1, caractérisé en ce que l'alcool contenant du carbonate de dialkyle provient de la séparation par distillation du mélange carbonate de dialkyle/alcool évacué de la transestérification en courant de tête de colonne en carbonate de dialkyle et un alcool contenant du carbonate de dialkyle.

3. Procédé selon revendication 2, caractérisé en ce que la séparation par distillation est réalisée a une pression de 4 à 15 bar, de préférence de 6 à 12 bar.

4. Procédé selon revendication 1, caractérisé en ce que l'alcool pur représente de 0,2 à 5 fois la proportion de carbonate de dialkyle contenue dans l'alcool.

5. Procédé selon revendication 1, caractérisé en ce que la transestérification est réalisée à une température de 40 à 150°C, de préférence de 45 à 130°C sous une pression de 0,5 à 2 bar, de préférence de 0,9 à 1,2 bar.

6. Procédé selon revendication 1, caractérisé en ce que l'on prépare le carbonate de diméthyle par transestérification du carbonate d'éthylèneglycol par le méthanol.

7. Procédé selon revendication 2, caractérisé en ce que le deuxième courant de tête de colonne obtenu à la séparation par distillation du mélange carbonate de dialkyle/alcool évacué de la transestérification en courant de tête de colonne est recyclé en proportion de 30 à 80 % en volume, de préférence de 40 à 70 % en volume, à la transestérification, et le reste est envoyé à une autre distillation dont le produit de pied de colonne consiste essentiellement en l'alcool et est recyclé à la partie inférieure de la colonne de transestérification, cependant que le produit de tête de cette deuixème colonne est recyclé à la première colonne de séparation (II).

8. Procédé selon revendication 2, caractérisé en ce que l'on envoie à la colonne de séparation (II) un courant de carbonate de dialkyle/alcool provenant de la préparation d'un carbonate d'alkyle et d'aryle ou d'un carbonate de diaryle.

9. Procédé selon revendication 1, caractérisé en ce que le glycol brut (17) obtenu après passage des évaporateurs à courant descendant/couche mince (III) et (IV) est envoyé pour préparation par distillation de glycol pur à une colonne (VII) en tête de laquelle on évacue une fraction de produits volatils (18) qu'on recycle à la colonne de transestérification (I), au-dessous de la tête de colonne, par une évacuation latérale (22), on évacue le glycol purifié qui peut encore être purifié dans une opération complémentaire (IX), le produit de pied (19) de la colonne (VII) étant envoyé avec une partie du produit de pied de colonne (12) de la colonne (IV) à une concentration dans la colonne (VIII) dont le courant de tête de colonne (21) est recyclé à la colonne de glycol (VII) et dont le produit de pied (6), contenant les produits lourds et une partie du catalyseur, est rejeté.
